# NEW EUROPEAN PATENT SPECIFICATION

(11) **EP 1 761 221 B2**
(45) Date of publication and mention of the opposition decision: **21.12.2016**
(45) Mention of the grant of the patent: 13.03.2013
(21) Application number: 05744755.9
(22) Date of filing: 19.05.2005
(51) Int. Cl.: A61F 5/448, A61F 5/443

(54) **ATTACHMENT MECHANYSM FOR OSTOMY BAGS**
BEFESTIGUNGSMECHANISMUS FÜR OSTOMIE-BEUTEL
MECANISME D'ATTACHE DE POCHES POUR STOMIE

(30) Priority: 10.06.2004 GB 0412892
(43) Date of publication of application: 14.03.2007
(73) Proprietor: SecuriCare (Medical) Limited, Loudwater High Wycombe Buckinghamshire HP10 9QY (GB)
(72) Inventor: Smith, Rory, Crawly, West Sussex RH10 2TU (GB); Finn, Richard, Crawley, West Sussex RH10 2TU (GB); Bird, Paul, Crawley, West Sussex RH10 2TU (GB)
(74) Representative: Lock, Graham James
(86) International application number: PCT/GB2005/001915
(87) International publication number: WO 2005/120405

(56) References cited:
- EP-A- 0 317 326
- EP-A- 1 013 250
- GB-A- 2 151 482
- US-A- 3 528 420
- US-A- 4 610 676
- US-A- 4 872 869
- US-A- 5 814 033

## Description

The present invention relates to an attachment mechanism for ostomy bags and like drainage bags, and in particular to an attachment mechanism for such bags which allows attachment of the bag to a peristomal pad secured to a wearer of the bag.

Ostomy bags for receiving bodily waste from colostomy or ileostomy patients are well known and a problem with such bags relates to the attachment mechanism for securing the bag in the correct position against the body of a wearer of the bag. Two-piece ostomy bags are generally secured in position against the wearer by attachment of the bag to a peristomal pad secured on the body of the wearer. The problem with current attachment means relates stems from the fact that the attachment involves only one single step. Once the bag and pad are initially connected the is not possible to manoeuvre the bag to a more desired position. This is particularly a problem when the wearer secures the bag to the pad when in a standing position but finds that the bag causes discomfort and irritation when the wearer subsequently moves to a sitting or lying position.

Furthermore, many common attachment mechanisms have been found to be fairly weak and easy to break or damage without due care and attention.

The present invention sets out to provide an ostomy bag with an improved attachment mechansim that overcomes or at least alleviates the problems described above by providing a two-step attachment mechanism in which the bag and pad are initially fitted together allowing relative rotational movement therebetween and then secured once the bag is in a desired position relative to the pad.

The invention provides an ostomy bag as defined in claim 1.

Preferably, each attachment member includes a flat ring-shaped base member.

Preferably the inner rim of each attachment member forms a concentric lip. Preferably still, the lip of one attachment member has a recess formed therein in which to receive the lip of the other member in use.

Preferably, one attachment member includes a concentric flange upstanding from a surface thereof. The flange aids location of that attachment member in relation to the other attachment member.

Preferably, the locating surfaces are fixed in engagement with each other, in use, with adhesive. Preferably, one surface has at least one concentric liner that is released to expose the adhesive located thereunder.

The invention will now be described by way of example with reference to the accompanying diagrammatic drawings, in which :-
Figure 1 is a side section view of an attachment mechanism constructed in accordance with the present invention;
Figure 2 is a perspective view of an attachment mechanism of Figure 1;
Figure 3 is a segmented perspective view of the attachment mechanism of Figures 1 and 2; and
Figure 4 is a further segmented perspective view of the attachment mechanism of Figures 1 to 3.

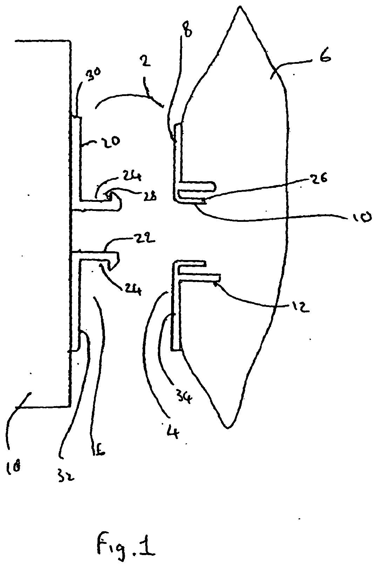

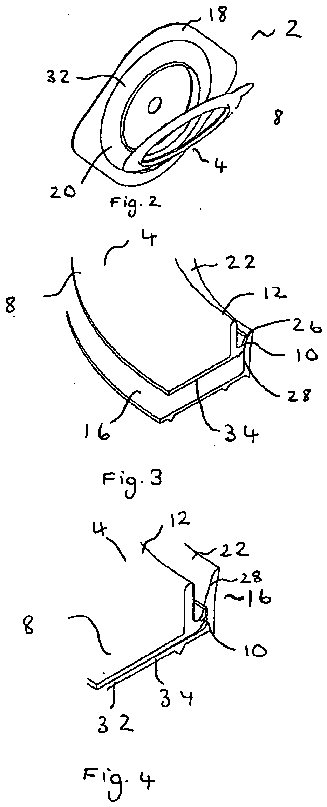

Referring first to Figures 1 and 2, the attachment mechanism 2 includes a first attachment member 4 attached to *inter alia* an ostomy bag 6 that is secured to the body of a wearer (not shown). The first attachment member 4 comprises a flat ring-shaped base 8, the inner rim of which is bent upwardly to form a concentric lip seal 10. A concentric flange 12 upstands from the base 8 next to the lip seal 10. The depth of the flange 12 is greater than that of the lip 10.

The attachment mechanism 2 further includes a second attachment member 16 attached to *inter alia* a peristomal pad 18 . The second attachment member 16 has a base 20 of similar shape and dimension as the first attachment member 4. The inner rim of the second attachment member 16 has an outwardly protruding lip 22. In the embodiment shown, the depth of the lip 22 is slightly greater than the depth of the flange 12 of the first attachment member 4 such that, when the base 8 of the first attachment member 4 is placed upon the base 20 of the second attachment member 16, the flange 12 and lip 22 are equal in height (as can be seen best in Figure 4).

The lip 22 of the second attachment member 16 has an inner recess 24 in a bottom portion thereof. In the embodiment shown, the depth of the recess 24 is slightly greater than the depth of the lip seal 10 of the first attachment member 4 such that, when the base 8 of the first attachment member 4 is placed upon the base 20 of the second attachment member 16, the lip seal 10 fits within the recess 24 such that a top edge 26 of the lip seal 10 abuts against the top inside edge 28 of the recess 24.

As can be seen in the Figure 4, the attachment members 4, 16 can be located together and retained in position to prevent lateral movement of the attachment members 4, 16 relative to each other, while still allowing relative rotational movement between the attachment members 4, 16. Consequently, the ostomy bag 6 and the peristomal pad 18 can be initially attached together via linking of the first and second attachment members 4, 16 while allowing rotational movement of the ostomy bag 6 with respect to the peristomal pad 18 such that the wearer can move the ostomy bag 6 to a location that is comfortable notwithstanding the position of the wearer.

The base 20 of the second attachment member 16, extending from the lip 22 to the outer rim 30 provides a smooth flat surface 32 on which adhesive (not shown) can be applied. The adhesive is initially covered by one more concentric release liners (also not shown).

Once the ostomy bag 6 and peristomal pad 18 have been attached via the attachment members 4, 16, and the ostomy bag 6 has been manouvered to a comfortable position, the release liners can be removed to the bottom surface 34 of the first attachment member 4 to be secured to the surface 32 of the second attachment member 16 by the adhesive. Once adhered together, relative rotational movement between the attachment members 4, 16 is prevented.

The combination of a mechanical and adhesive attachment ensures that the resulting link between the first and second attachment members 4, 16 is very strong.

The above described embodiment has been given by way of example only, and the skilled reader will naturally appreciate that many variations could be made thereto without departing from the scope of the present invention.

## Claims

1. An ostomy bag (6) including an attachment mechanism for attachment to a peristomal pad (18), the attachment mechanism comprising first and second attachment members (4, 16) associated with the bag and pad respectively, each attachment member (4, 16) having a respective locating surface (34, 32) and one locating surface having at least one release liner which is releasable to expose adhesive located thereunder,
**characterised in** the first and second attachment members (4, 16) being shaped and conformed to allow one attachment member (4, 16) to be located on and secured to the other attachment member in such a way as to allow only rotational movement of the attachment members (4, 16) in respect of each other when the locating surfaces (34, 32) are not engaged, whereby on removal of the or each release liner from the respective attachment members the rotational movement is prevented by engagement of the locating surfaces (34, 32) of the first and second attachment members.

2. An ostomy bag (6) according to claim 1, wherein each attachment member (4, 16) includes a flat ring-shaped base member (8, 20).

3. An ostomy bag (6) according to claim 2, wherein the inner rim of each attachment member forms a concentric lip (10, 22).

4. An ostomy bag (6) according to claim 3, wherein the lip (22) of one attachment member (16) has a recess (24) formed therein in which to receive the lip (10) of the other member (4) in use.

5. An ostomy bag (6) according to any one of claims 1 to 4, wherein one attachment member (4) includes a concentric flange (12) upstanding from a surface (8) thereof to aid location of that attachment member (4) in relation to the other attachment member (16).

6. An ostomy bag (6) according to any one of claims 1 to 5, wherein the locating surfaces (34, 32) are fixed in engagement with each other, in use, by the adhesive.

## Patentansprüche

1. Ein Ostomie-Beutel, einen Befestigungsmechanismus beinhaltend zum Befestigen an einer peristomalen Einlage (18), wobei der Befestigungsmechanismus erste und zweite Befestigungselemente (4, 16) umfasst, zugehörig zu dem Beutel beziehungsweise der Einlage, wobei jedes Befestigungselement (4, 16) eine entsprechende Anschlagfläche (34, 32) hat, und wobei eine Anschlagfläche wenigstens ein Trennpapier hat, das ablösbar ist, um darunter befindlichen Kleber freizulegen,
**gekennzeichnet durch** die ersten und zweiten Befestigungselemente (4, 16), die geformt und angepasst sind, um einem Befestigungselement (4, 16) zu ermöglichen, an dem anderen Befestigungselement in der Weise angeordnet und gesichert zu sein, nur Rotationsbewegung der Befestigungselemente (4, 16) in Bezug aufeinander, wenn die Anschlagflächen (34, 32) nicht verbunden sind, zu ermöglichen, wobei bei Entfernung des oder jedes Trennpapiers von den entsprechenden Befestigungselementen die Rotationsbewegung verhindert wird **durch** Verbindung der Anschlagflächen (34, 32) der ersten und zweiten Befestigungselemente.

2. Ein Ostomie-Beutel (6) nach Anspruch 1, wobei jedes Befestigungselement (4, 16) ein flaches ringförmiges Basiselement (8, 20) beinhaltet.

3. Ein Ostomie-Beutel (6) nach Anspruch 2, wobei der Innenrand jedes Befestigungselements eine konzentrische Lippe (10, 22) formt.

4. Ein Ostomie-Beutel (6) nach Anspruch 3, wobei die Lippe (22) des einen Befestigungselements (16) eine darin geformte Mulde (24) aufweist, in die, im Gebrauch, die Lippe (10) des anderen Elements (4) aufzunehmen ist.

5. Ein Ostomie-Beutel (6) nach einem der Ansprüche 1 bis 4, wobei ein Befestigungselement (4) eine von dessen Oberfläche abstehende konzentrische Kante (12) beinhaltet, um die Positionierung dieses Befestigungselements (4) in Bezug auf das andere Befestigungselement (16) zu unterstützen.

6. Ein Ostomie-Beutel (6) nach einem der Ansprüche 1 bis 5, wobei die Anschlagflächen (34, 32) im Gebrauch bei Verbindung miteinander durch den Kleber fixiert sind.

## Revendications

1. Poche de stomie (6) comprenant un mécanisme de fixation pour fixer un tampon péristomal (18), le mécanisme de fixation comprenant des premier et second éléments de fixation (4, 16) associés à la poche et au tampon respectivement, chaque élément de fixation (4, 16) ayant une surface de positionnement respective (34, 32) et une surface de positionnement ayant au moins un revêtement amovible qui est amovible pour exposer l'adhésif situé en dessous,
**caractérisé en ce que** les premier et second éléments de fixation (4, 16) sont façonnés et formés de façon à permettre à un élément de fixation (4, 16) d'être placé sur, et fixé à, l'autre élément de fixation afin de permettre seulement un mouvement rotatif des éléments de fixation (4, 16) les uns par rapport aux autres, quand les surfaces de positionnement (34, 32) ne sont pas en prise, moyennant quoi, lors de l'enlèvement du ou de chaque revêtement des éléments de fixation respectifs, le mouvement rotatif est empêché par la mise en prise des surfaces de positionnement (34, 32) des premier et second éléments de fixation.

2. Poche de stomie (6) selon la revendication 1, dans laquelle chaque élément de fixation (4, 16) comprend un élément à base annulaire plat (8, 20).

3. Poche de stomie (6) selon la revendication 2, dans laquelle la garniture interne de chaque élément de fixation forme un rebord concentrique (10, 22)

4. Poche de stomie (6) selon la revendication 3, dans laquelle le rebord (22) d'un élément de fixation (16) a une cavité (24) formée à l'intérieur, permettant de recevoir le rebord (10) de l'autre élément (4) utilisé.

5. Poche de stomie (6) selon l'une quelconque des revendications 1 à 4, dans laquelle un élément de fixation (4) comprend une bride concentrique (12) se dressant à partir d'une surface (8) de celle-ci pour aider à positionner cet élément de fixation (4) par rapport à l'autre élément de fixation (16).

6. Poche de stomie (6) selon l'une quelconque des revendications 1 à 5, dans laquelle les surfaces de positionnement (34, 32) sont fixées en prise les unes avec les autres, en cours d'utilisation, par l'adhésif.
